# EUROPEAN PATENT SPECIFICATION

(11) **EP 1 411 984 B1**
(45) Date of publication and mention of the grant of the patent: **07.05.2008**
(21) Application number: 02745167.3
(22) Date of filing: 24.05.2002
(51) Int. Cl.: A61K 31/44, A61K 45/06, A61P 35/00

(54) **COMBINATION MEDICAMENT FOR TREATMENT OF NEOPLASTIC DISEASES WHICH CONTAINS CYANOGUANIDINE IKK INHIBITORS AND A SECOND ANTI-NEOPLASTIC DRUG**
ZUSAMMENSETZUNGEN ZUR BEHANDLUNG VON NEOPLASTISCHEN KRANKHEITEN WELCHE EINEN CYANOGUANIDINE IKK INHIBITOR UND EIN ZWEITES ANTINEOPLASTISCHES ARZNEIMITTEL ENTHALTEN
MEDICAMENTS COMBINATOIRES UTILISES DANS LE TRAITEMENT DE MALADIES NEOPLASIQUES QUI CONTIENNENT UN CYANOGUANIDINE INHIBITEUR DE IKK ET UN DEUXIEME COMPOSE ANTI-NEOPLASIQUE

(30) Priority: 24.05.2001 US 292928 P
(43) Date of publication of application: 28.04.2004
(73) Proprietor: LEO PHARMA A/S, 2750 Ballerup (DK)
(72) Inventor: BINDERUP, Lise, DK-2630 Taastrup (DK); BRAMM, Erik, DK-2610 Roedovre (DK); HJARNAA, Pernille-Julia, Vig, DK-3060 Espergaerde (DK); HAMBERG, Karin, Jexner, DK-2840 Holte (DK)
(74) Representative: Duckworth, Timothy John
(86) International application number: PCT/DK2002/000351
(87) International publication number: WO 2002/094322

(56) References cited:
- WO-A-94/06770
- WO-A-98/54141
- WO-A-98/54142
- WO-A-98/54143
- WO-A-98/54144
- WO-A-98/54145
- A.SVENSSON E.A.: "CHS 828 inhibits neuroblastoma growth in mice alone and in combination with antiangiogenic drugs" PEDIATRIC RESEARCH, vol. 51, no. 5, 2002, pages 607-611, XP008007245

## Description

### FIELD OF INVENTION

The invention relates to the use of IKK inhibitors for the manufacture of anti-neoplastic medicaments to prevent the development of resistance of cancer cells to other anti-neoplastic drugs.

### BACKGROUND OF THE INVENTION

Neoplastic diseases are characterised by autonomous growth of cells. Neoplastic diseases may be benign, i.e. the growth is contained and does not spread to other organs or parts of the body. Neoplastic diseases may also be malignant where the growth spreads to other organs or parts of the body by infiltration or metastases. Malignant neoplastic diseases are also known as cancer.

Patients with neoplastic diseases are conveniently treated by surgery, ionising radiation, medication, or a combination thereof. Several types of medicaments or drugs for the treatment of neoplastic diseases are known, and one way of classifying these medicaments is suggested in Abeloff et al (Eds.), Clinical Oncology, Churchill Livingston Inc., New York, 1995 . Medicaments for treatment of neoplastic diseases (anti-neoplastic drugs) may conveniently be classified as chemotherapeutic agents, hormonal agents or biological response modifiers.

Chemotherapeutic agents may be further classified according to the mechanism whereby they effect their response e.g. as S-triazine derivatives such as altretamine; as ezymes such as asparaginase; as antibiotic agents such as bleomycin, dactinomycin, daunorubicin, doxorubicin, idarubicin, mitomycin and plicamycin; alkylating agents such as busulfan, carboplatin, carmustine, chlorambucil, cisplatin, cyclophosphamid, dacarbazine, ifosfamide, lomustine, mechlorethamine, melphalan, procarbazine and thiotepa; as antimetabolites such as cladribine, cytarabine, floxuridine, fludarabine, fluoruracil, hydroxyurea, mercaptopurine, methotrexate, pentostatin and thioguanine; and as antimitotic agents such as etoposide, paclitaxel, teniposide, vinblastine and vincristine.

Hormonal agents may be further classified according to the mechanism whereby they effect their response, e.g. as aromatase inhibitors such as aminoglutethimide; as antiestrogens such as tamoxifen, formestan and letrozol; and as antiandrogen such as flutamide.

Biological response modifiers may be further classified according to the mechanism whereby they effect their response, e.g. as lymphokines such as aldesleukin; as interferon such as interferon-α; and as growth factors such as erythropoietin, filgrastim and sagramostim.

A number of medicaments do not fall naturally within these classifications. Examples of such medicaments are differentiating agents such as all-trans retinoic acid, immunoregulators such as levamisole, and vitamin D analogues, such as seocalcitol.

Other types of medicaments based on e.g monoclonal antibodies, tumour necrosis factor, gene therapy and angiogenesis inhibitors have been suggested for treatment of neoplastic diseases, but they are still in the exploratory phase.

Despite the classification indicated above, many anti-neoplastic drugs and ionising radiation seem to rely on a common principal mechanism, namely apoptosis, by which they effect their responses. Apparently, the direct effect of anti-neoplastic drugs and ionising radiation may be of less importance to the final results of the therapy than their ability to induce apoptosis in the cells. [Friesen, Nature Medicine, 2, 574-577, 1996; Fisher, Cell, 78, 539-542, 1994]. Apoptosis is a genetically encoded cell death programme characterised by an "active decision" by the cell based on information from its environment, its own internal metabolism, its developmental history, etc to die. Unlike cells undergoing necrosis, cells stimulated to enter apoptosis are often capable of survival, but opt to die for the good of the whole organism. Apoptosis is also different from necrosis in that necrosis is often associated with traumatised tissue and cell bursts, whereas the cells condense in the course of apoptosis, and are degraded intracellularly in a controlled manner [Tran, Science and Medicine, 6, 18-27, 1999; Williams, Trends Cell Biol., 2, 263-267, 1992].

Unfortunately, neoplastic cells are very effective in developing biochemical mechanisms that allow cellular resistance to medicaments or ionising radiation. In fact, resistance is a common clinical problem in the therapy of neoplastic diseases [Cun-Yu Wang, Nature Medicine, 5,412-417, 1999]. In order to overcome this resistance, therapy generally involves more than one medicament or combinations of ionising radiation and medicaments. Several types of resistance are known, e.g. enhanced drug metabolism, altered drug accumulation, drug target amplification and repair of damaged targets. Resistance to apoptosis is another type of multi-drug resistance, that likely explains a significant proportion of treatment failures [Fisher, Cell, 78, 539-542, 1994]. For convenience, the terms "medicament" and "drug" are used interchangeably, and are intended to indicate the same.

At the cellular level it is well recognised that Nuclear Factor κB (NFκB) plays a pivotal role in apoptosis and resistance to apoptosis. It is also described that an NFκB inhibitor, IκB, and an IκB kinase complex, IKK, control the level of activated NFκB [Levkau, 1, 227-233, 1999; Wang, Science, 274, 784-787, 196; Madrid, Molecular and Cellular Biology, 5, 1626-1638, 2000]. Accordingly, the NFκB-IκB-IKK system has been suggested as a target in the treatment of neoplastic diseases.

Cusack, Cancer Research, 60, 2323-2330, 2000 and Wang, Nature Medicine 5, 412-417, 1999 teach that a particular chemotherapeutic agent, namely the topoisomerase I inhibitor 7-ethyl-10-[4-(1-piperidino)-1-piperidino]-carbonyloxycamptothecin (CPT-11) activates NFκB activity in cells to induce resistance toward itself, and that an adenoviral transfer of an IκB, IκBα, to inhibit NFκB promotes chemosensitivity to treatment with CPT-11.

WO98/37228 teaches that an agent which decreases IKK activity or that alters the association of IKK and IκB can be useful for allowing apoptosis to occur in a tumour cell by increasing the level of unphosphorylated IκB, which can bind to NFκB and decrease the level of active NFκB in the tumour cell.

Rossi, Nature, 403, 103-108, 2000 teaches that cyclopentenone prostaglandins inhibit IκB kinase, and that this makes cyclopentenone prostaglandins potentially valuable in the treatment of cancers, inflammation and viral infections.

### SUMMARY OF THE INVENTION

It has surprisingly been found that certain cyanoguanidine compounds are capable of modulating the level of activated NFκB through inhibition of the IκB kinase complex (abbreviated IKK in the following), thereby preventing resistance to the apoptosis effected by other anti-neoplastic drugs and ionising radiation. Cyanoguanidine compounds are thus able to increase the effect of other anti-neoplastic treatments. Synergistic effects may therefore be obtained in the treatment of patients with neoplastic diseases by combining treatment with cyanoguanidine compounds with other types of anti-neoplastic treatment, e.g. treatment with chemotherapeutic agents, hormonal agents, angiogenesis inhibitors and differentiating agents.

It is a well-recognised problem with all anti-neoplastic therapies that they give rise to severe adverse effects such as nausea, hair loss, myelosuppression etc because of their toxicity. Due to the seriousness of the prognosis for neoplastic diseases, a high level of adverse effects can be accepted if the therapy appears curative. However, synergistically increasing the effect of traditional anti-neoplastic treatments by concomitantly administering cyanoguanidine compounds to patients, may allow for a reduction of the doses thus providing more effective and less toxic treatments with fewer adverse effects.

Accordingly, the invention relates to the use of a cyanoguanidine IKK inhibitor compound of the general formula I 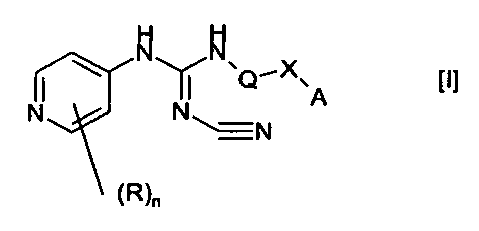 wherein
n is 0, 1 or 2;
each R independently represents halogen, trifluoromethyl, hydroxy, C₁₋₄ alkyl, C₁₋₄ alkoxy, C₁₋₄ alkoxycarbonyl, nitro, sulfo, cyano, amino or carboxy groups;
Q is a straight or branched, saturated or unsaturated C₄₋₂₀ divalent hydrocarbon radical;
X is a bond, amino, O, S, carbonyl, carbonylamino, aminocarbonyl, oxycarbonyloxy, oxycarbonyl, carbonyloxy, aminocarbonyloxy, aminothiocarbonyloxy, oxycarbonylamino or oxythiocarbonylamino;
A is di-(C₁₋₄ alkoxy)phospinoyloxy, C₁₋₄ alkoxycarbonyl, C₁₋₄ alkoxycarbonylamino or C₃₋₁₂ carbocyclic ring optionally saturated with one or more R₁; R₁ being independently selected from the group consisting of halogen, trifluoromethyl, hydroxy, C₁₋₄ alkyl, C₁₋₄ alkoxy, C₁₋₄ alkoxycarbonyl, nitro, cyano, amino, sulfo, carboxy, carboxamido, sulfamoyl or C₁₋₄ hydroxyalkyl;
or a pharmaceutically acceptable salt or N-oxide thereof as a first anti-neoplastic drug for the preparation of a medicament for treatment of neoplastic diseases which, when administered simultaneously or sequentially with a second anti-neoplastic drug, is for prevention of the development of resistance of cancer cells to apoptosis that results from the activation of NFκB by said second anti-neoplastic drug. The present invention also provides a compound of the above general formula I for use as a first antineoplastic drug in the treatment of neoplastic diseases, which treatment comprises administering simultaneously or sequentially said compound and a second anti-neoplastic drug and is for prevention of resistance of cancer cells to apoptosis that results from the activation of NFκB by said second anti-neoplastic drug.

### DETAILED DESCRIPTION OF THE INVENTION

The term "alkyl" is intended to indicate a univalent radical derived from straight, branched or cyclic alkane by removing a hydrogen atom from any carbon atom. The term includes the subclasses primary, secondary and tertiary alkyl, such as methyl, ethyl, n-propyl, isopropyl, n-butyl, isobutyl, tert.-butyl, isopentyl and isohexyl.

The term "divalent hydrocarbon radical" is intended to include straight or branched, saturated or unsaturated carbon chains, e.g. alkylene, alkenylene or alkynylene.

The term "alkoxy" is intended to indicate a radical of formula OR', wherein R' is alkyl as defined above, e.g. methoxy, ethoxy, propoxy, butoxy, etc.

The term "alkoxycarbonyl" is intended to indicate a radical of formula -COOR' wherein R' is alkyl as defined above, e.g. methoxycarbonyl, ethoxycabonyl, n-propoxycarbonyl, isopropoxycarbonyl, etc.

The term "carbocyclic ring" is intended to include radicals of saturated or unsaturated rings, optionally fused bicyclic rings, e.g. cyclopropyl, cyclopentyl, cyclopentenyl, cyclohexyl, cyclohexenyl, phenyl, nafthyl, dihydronafthyl, pentalenyl, indanyl and indenyl.

The term "hetero carbocyclic ring" is intended to include radicals of saturated or unsaturated heterocyclic rings, optionally fused bicyclic rings, with one or more heteroatoms selected from O, S and N, e.g. pyridyl, tetrazolyl, thiazolyl, imidazolyl, pyrazolyl, oxazolyl, isoxazolyl, thienyl, pyrazinyl, pyranyl, isothiazolyl, benzimidazolyl and benzofuranyl, pyrrolyl, furanyl, pyrazolyl, pyrrolidinyl, pyridyl, pyrimidinyl, tetrahydrothieyl, tetrahydrofuranyl, tetrahydropyranyl, piperidyl, quinolyl, isoquinolyl, 1,2-dihydroquinolyl, etc.

The term "halogen" is inteded to indicate fluoro, chloro, bromo or iodo.

The term "pharmaceutically acceptable salt" is intended to indicate salts prepared by reacting a compound of formula I comprising a basic group with a suitable inorganic or organic acid, e.g. hydrochloric, hydrobromic, hydroiodic, sulfuric, nitric, acetic, phosphoric, lactic, maleic, phthalic, citric, propionic, benzoic, glutaric, gluconic, methanesulfonic, salicylic, succinic, tartaric, toluenesulfonic, sulfamic or fumaric acid. Pharmaceutically acceptable salts of compounds of formula I comprising an acidic group may be prepared by reaction with a suitable base such as sodium hydroxide, potassium hydroxide, ammonia or the like.

The term "N-oxide" is intended to indicate e.g. pyridyl N-oxide derivatives of the compounds of the invention. Such compounds may be prepared by oxidation of the pyridyl N by a suitable oxidising agent, e.g. 3-chloro-perbenzoic acid in an inert solvent, e.g. dichlormethan.

The term "resistance" is intended to indicate a reduced sensitivity to a given treatment. Sensitivity can be defined in terms of IC₅₀, which indicates the amount or concentration of a given treatment or ionising radiation, which is lethal to 50% of the cells. An increase in IC₅₀ signifies a reduced sensitivity to a given therapy, and the cells are termed "resistant" if IC₅₀ increases by a factor of 10 or more, e.g. by a factor of 20-50. This definition is of particular relevance for in vitro studies, but of less relevance for in vivo studies, not to mention treatment of human beings. For in vivo studies and in human therapy a more feasible definition of resistance may be expressed as the overall failure of treatment, defined as progressing neoplastic diseases in a patient who previously responded to treatment. Progressing neoplastic diseases may be defined as ≥25% increase in the size of one or more lesions or the appearance of new lesions [WHO Handbook for reporting results of cancer treatment, Publication No.48, Geneva, WHO, 1979].

The term "modulate" when used in relation to levels of activated NFκB means that the level of activated NFκB is increased or decreased compared to the level present in the absence of a compound of the general formula I. The level of activated NKκB is preferably decreased by the compound of formula I.

The term "effective amount" is intended to indicate the amount which is required to confer a therapeutic effect to the treated patient, and is typically determined on the basis of the age, surface area, body weight, the desired effect, and condition of the patient.

The term "excipient" when used herein is intended to indicate all substances in a pharmaceutical formulation which are not active ingredients, such as e.g. carriers, binders, lubricants, thickeners, surface active agents, preservatives, emulsifiers, buffers, flavouring agents or colourants.

The term "cyanoguanidine compound" is intended to indicate a compound comprising the structure shown in formula II

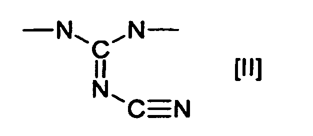

The term comprises, but is not limited to compounds of formula I, e.g. cyanoguanidine compounds disclosed in WO 00/61559, WO 00/61561, WO 00/76516, WO 00/76517 and PCTDK01/00750 are also included in this definition.

Preferred compounds of formula I are those wherein A is a phenyl, optionally substituted with a substituent selected from the group consisting of halogen, trifluoromethyl, hydroxy, C₁₋₄ alkyl, C₁₋₄ alkoxy, C₁₋₄ alkoxycarbonyl, nitro, cyano, amino, carboxy, carboxamido, sulfamoyl or C₁₋₄ hydroxyalkyl.

In another preferred embodiment of the invention A is an optionally substituted tetrahydropyranyl.

In a further preferred embodiment of the invention X is O.

In a further preferred embodiment of the invention X is amino.

In a still further preferred embodiment of the invention Q is a C₄₋₁₂ divalent hydrocarbon radical.

In a still further preferred embodiment of the invention n is 0 or n is 1, R being C₁₋₄ alkoxy.

In a still further preferred embodiment of the invention the compound of formula I is selected from the group consisting of
N-cyano-N'-(6-(4-chlorophenoxy)hexyl)-N"-(4-pyridyl)guanidine
N-cyano-N'-(7-phenoxyheptyl)-N"-(4-pyridyl) guanidine
N-(12-(tert-butoxycarbonylamino)dodecanyl)-N'-cyano-N"-(4-pyridyl) guanidine
N-cyano-N'-(11-(tetrahydropyran-2-yloxy)-undecanyl-N"-(4-pyridyl) guanidine
N-cyano-N'-(6-(2-methoxyphenoxy)hexyl)-N"-(4-pyridyl) guanidine
N-(6-(2,4,5-trichlorophenoxy)hexyl)-N'-cyano-N"-(4-pyridyl) guanidine
N-(6-(1-chlorophenoxy)hexyl))-N'-cyano-N"-(4-pyridyl) guanidine

Compounds of the general formula I contain asymmetric carbon atoms as well as carbon-carbon double bonds, which allow for isomeric forms. It is understood that the present invention relates to any tautomer, diastereomer and optical isomer expressed by the general formula I, either in pure from or as mixtures thereof.

Compounds of formula I are known from the literature and methods of their synthesis have previously been disclosed [EP660 823, WO 98/54141, WO 98/54143 and WO 98/54145]. While the compounds have been suggested for cancer therapy, no indication in these publications has been offered as to the mechanisms of action to which they owe their effect nor to any synergistic effect between cyanoguanidine compounds and other types of treatment for neoplastic diseases.

NFκB is a member of the Rel family of transcription factors, which are ubiquitous in animal cells. Rel proteins can form dimers, the most common of which is designated NFκB. NFκB is a p50/p65 heterodimer which can activate transcription of genes containing the appropriate κB binding site. In non-stimulated cells, NFκB is maintained in the cytoplasm by an interaction with NFκB inhibiting proteins, the IκBs. In response to cell stimulation by e.g. anti-neoplastic drugs or ionising radiation an IκB kinase complex (IKK) is rapidly activated and phosporylates two serine residues in the NFκB binding domain of IxB. The phophorylated IκB is then degraded by a 26S proteasome whereas NFκB is spared from degradation and translocates into the nucleus [Wang, Science, 274, 784-787, 1996, Cusak, Cancer Research, 60, 2323-2330, 2000; Karin, Immunology, 12, 2000, 85-98]. NFκB is thus always present in the cell, but in an inactivated form in non-stimulated cells. After translocation into the nucleus NFκB induces inter alia the anti-apoptotic genes c-IAP1, c-IAP2, TRAF1, TRAF2, Bfl-1/A1, Bcl-X_{L} and Mn-SOD [Platel, Oncogene, 19, 2000, 4159-4169], which bring about resistance in the cells to apoptosis. This effect is referred to as the anti-apoptotic effect of NFκB, and the effect may be quantified by measuring the expression of compounds encoded by any of said genes, by any suitable means known in the art, in the presence and absence of compounds modulating the level of activated NKκB. Any compound capable of reducing the transcription of said genes to a level less than 50%, e.g. less than 30%, such as less than 20% of the level in the absence of said compound is said to reduce the antiapoptotic effect of NKκB. Anti-neoplastic drugs and ionising radiation thus induce resistance in the cells to the treatments, which render them ineffective. Accordingly, activated NFκB is a key factor in induced resistance in e.g. cancer cells to anti-neoplastic drugs and/or to ionising radiation. This is further supported by the fact that constitutively activated NFκB is found in cells from resistant cancer tumours [Patel, Oncogene, 19, 4159-4169, 2000].

In cells not exhibiting resistance to anti-neoplastic treatments, a reduction of the level of activated NFκB in the cell, e.g. by controlling the activity of IKK, will reduce the expression levels of genes encoding for anti-apoptotic factors inducing apoptosis in the cells [Schwartz, Surgical Oncology.8, 1999, 143-153].

Accordingly, a reduction of the level of activated NFκB, e.g. by cyanoguanidine effected IKK inhibition, has therapeutic value in the treatment of neoplastic diseases at at least two levels. Firstly, treatment with cyanoguanide compounds will induce apoptosis in the cells so that cyanoguanidine compounds themselves may effect death of neoplastic cells. Secondly, it will increase the effect of other anti-neoplastic treatments by preventing the resistance to the treatment normally induced by these treatments.

IκB is non-covalently bound to NFκB and masks its nuclear localisation signal, thereby preventing translocation into the nucleus. Various IκB have been identified and e.g. IκBα and IκBβ are expressed in most cells where they bind to p65 Rel proteins, i.e. NFκB. Different IκB are phosphorylated by different factors allowing activation of NFκB in response to different stimuli.

The IκB kinase complex consist of three subunits, namely IKKα, IKKβ and IKKγ, with a combined molecular weight of 900 kDa. IKKα and IKKβ both exhibit IκB kinase activity and phophorylate IκB, whereas IKKγ is a regulatory subunit. IKKα is 85 kDa protein and IKKβ is a 87 kDa protein, and the two subunits show a large degree of homology. Whereas both IKKα and IKKβ are catalytically active, it has surprisingly been shown that only IKKβ is essential for IKK phosphorylation of IκB.

The term "inhibitor" when used herein is intended to indicate a compound capable of decreasing or even blocking the activity of a given enzyme system, e.g. IKK, towards a given substrate. Several methods to identify compounds capable of inhibiting IKK are known to the person skilled in the art. Such a method may make the form of an assay which may comprise e.g. isolated IKK or subunits thereof exposed to compounds suspected to modulate the IKK activity. Methods of obtaining isolated IKK or subunits thereof are known in the art. They comprise e.g. immunoprecipitation or expression of IKK or subunits thereof in a suitably selected host cell, e.g. as disclosed in WO98/37228. The IKK activity may conveniently be measured by determining phosphorylation of e.g. IκB, either directly or by using antibodies against phophorylated IκB. Other suitable substrates for IKK may also be used. The result from such an experiment conducted in the presence of a given compound is compared to a similar experiment conducted in the absence of said compound. The assay may also be a cellular assay in which cells expressing IKK or subunits thereof are exposed to compounds suspected of modulating IKK activity. The cells in a cellular assay may be manipulated to enhance the expression level of IKK or subunits thereof. Methods for manipulating the expression level of proteins in cells are known in the art examples of which are genetic manipulation, classic mutation and selection. Following exposure for an appropriate amount of time, the cells may be collected, lysed, and the IKK immunoprecipitated using an appropriate antibody. A substrate, I_{K}B or another suitable substrate may then be added to the immunocomplex, and its ability to phosphorylate the substrate may be determined as described above, and the result compared to the result from a similar experiment performed in the absence of the compound under investigation.

The ability of compounds to modulate the activity of IKK or subunits thereof in a cellular assay may also be determined without disrupting the cells through lysis. After exposure of the cells to a compound for an appropriate amount of time, the secretion from the cells of e.g. specific cytokines regulated by NFκB may be measured. Examples of such specific cytokines are tumour necrosis factor α (TNFα) and interleukin 1 (IL1). The result is compared to results from similar experiments conducted in the absence of the compound under investigation. The specific compounds regulated by NFκB and secreted by the cell may vary between different types of cells. A person skilled in the art are capable of choosing a relevant compound which to measure for a given cell system.

Regardless of how an IKK assay is run, a compound which reduces the activity of either IKK or the level of activated NKκB to a level less than 50%, e.g. less than 30% or even less than 20% of the level in the absence of said compound is said to inhibit IKK.

The formulations used in the present invention, both for veterinary and for human medical use, comprise active ingredients in association with a pharmaceutically acceptable carrier(s) and optionally other therapeutic ingredient(s). The carrier(s) must be "acceptable" in the sense of being compatible with the other ingredients of the formulations and not deleterious to the recipient thereof.

Examples of anti-neoplastic drugs which may suitably be included in the composition as the second active compound are chemotherapeutic agents e.g. S-triazine derivatives such as altretamine; enzymes such as asparaginase; antibiotic agents such as bleomycin, dactinomycin, daunorubicin, doxorubicin, idarubicin, mitomycin and plicamycin; alkylating agents such as busulfan, carboplatin, carmustine, chlorambucil, cisplatin, cyclophosphamid, dacarbazine, ifosfamide, lomustine, mechlorethamine, melphalan, procarbazine and thiotepa; antimetabolites such as cladribine, cytarabine, floxuridine, fludarabine, fluoruracil, hydroxyurea, mercaptopurine, methotrexate, pentostatin and thioguanine; and antimitotic agents such as etoposide, paclitaxel, teniposide, vinblasine and vincristine; hormonal agents e.g. aromatase inhibitors such as aminoglutethimide; antiestrogens such as tamoxifen, formestan and letrozol; as antiandrogen such as flutamide; and angiogenesis inhibitors.

The formulations include e.g. those in a form suitable for oral, ophthalmic, rectal, parenteral (including subcutaneous, intramuscular, interperitoneal, intraarticular and intravenous), transdermal, and topical, nasal or buccal administration.

By the term "dosage unit" is meant a unitary, i.e. a single dose which is capable of being administered to a patient, and which may be readily handled and packed, remaining as a physically and chemically stable unit dose comprising either the active material as such or a mixture of it with solid or liquid pharmaceutical diluents or carriers.

The formulations may conveniently be presented in dosage unit form and may be prepared by any of the methods well known in the art of pharmacy, e.g. as disclosed in Remington, The Science and Practise of Pharmacy, 20th ed., 2000. All methods include the step of bringing the active ingredient into association with the carrier, which constitutes one or more accessory ingredients. In general, the formulations are prepared by uniformly and intimately bringing the active ingredient into association with a liquid carrier or a finely divided solid carrier or both, and then, if necessary, shaping the product into the desired formulation.

Formulations suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid, such as ethanol or glycerol; or in the form of an oil-in-water emulsion or a water-in-oil emulsion. Such oils may be edible oils, such as e.g. cottonseed oil, sesame oil, coconut oil or peanut oil. Suitable dispersing or suspending agents for aqueous suspensions include synthetic or natural gums such as tragacanth, alginate, acacia, dextran, sodium carboxymethylcellulose, gelatin, methylcellulose and polyvinylpyrrolidon. The active ingredients may also be administered in the form of a bolus, electuary or paste.

A tablet may be made by compressing or moulding the active ingredient optionally with one or more accessory ingredients. Compressed tablets may be prepared by compressing, in a suitable machine, the active ingredient(s) in a free-flowing form such as a powder or granules, optionally mixed by a binder, such as e.g. lactose, glucose, starch, gelatine, acacia gum, tragacanth gum, sodium alginate, carboxymethylcellulose, polyethylene glycol, waxes or the like; a lubricant such as e.g. sodium oleate, sodium stearate, magnesium steatrate, sodium benzoate, sodium acetate, sodium chloride or the like; a disintegrating agent such as e.g. starch, methyl cellulose, agar, bentonite, xanthan gum or the like or dispersing agent. Moulded tablets may be made by moulding, in a suitable machine, a mixture of the powdered active ingredient and suitable carrier moistened with an inert liquid diluent.

Formulations for rectal administration, e.g. injection or infusion, may be in the form of a suppository incorporating the active ingredients and a carrier, or in the form of an enema.

Formulations suitable for parenteral administration conveniently comprise a sterile oily or aqueous preparation of the active ingredients, which is preferably isotonic with the blood of the recipient, e.g. isotonic saline, isotonic glucose solution or buffer solution. Liposomale formulations may also be used to present the active ingredients for parenteral administration. The formulation may be conveniently sterilised by for instance filtration through a bacteria retaining filter, addition of sterilising agent to the formulation, irradiation of the formulation or heating of the formulation.

Alternatively, the composition may be provided as a sterile, solid preparation, e.g. a freeze-dried powder, which is readily dissolved in a sterile media just prior to use.

Transdermal formulations may be in the form of a plaster.

Formulations suitable for ophthalmic administration may be in the form of a sterile aqueous preparation of the active ingredients, which may be in microcrystalline form, for example, in the form of an aqueous microcrystalline suspension. Liposomal formulations or biodegradable polymer systems may also be used to present the active ingredient for ophthalmic administration.

Formulations suitable for topical or ophthalmic administration include liquid or semi-liquid preparations such as liniments, lotions, gels, applicants, oil-in-water or water-in-oil emulsions such as creams, ointments or pastes; or solutions or suspensions such as drops.

In addition to the aforementioned ingredients, the formulations of a cyanoguanidine IKK inhibitor compound of formula I and of a second anti-neoplastic drug may include one or more additional ingredients such as diluents, buffers, flavouring agents, colourant, surface active agents, thickeners, preservatives, e.g. methyl hydroxybenzoate (including anti-oxidants), emulsifying agents and the like.

In addition to the formulations described previously, the compositions can also be formulated as a depot preparation. Such long-acting formulations may be administered by implantation (e.g. subcuteneously or intramuscular) or by intramuscular injection. Thus, for example, the components of the present invention may be formulated with suitable polymeric or hydrophobic materials (for example as an emulsion in a pharmaceutically acceptable oil), or ion exchange resin, or as sparingly soluble derivatives, for example, as a sparingly soluble salt.

In the systemic treatment using the present invention daily doses of from 0.001-200 mg per kilogram body weight, preferably from 0.002-50 mg/kg of mammal body weight, for example 0.003-10 mg/kg of a cyanoguanidine IKK inhibitor compound of formula I and a second anti-neoplastic drug are administered, typically corresponding to a daily dose for an adult human of from 0.2 to 1500 mg of each compound. In the topical treatment of dermatological disorders, ointments, creams or lotions containing from 0.1-750 mg/g, and preferably from 0.1-500 mg/g, of a cyanoguanidine IKK inhibitor compound of formula I and a second anti-neoplastic drug are administered. For topical use in ophthalmology ointments, drops or gels containing from 0.1-750 mg/g, and preferably from 0.1-500 mg/g, of a cyanoguanidine IKK inhibitor compound of formula I and a second anti-neoplastic drug are administered. The oral compositions are formulated, preferably as tablets, capsules, or drops, containing from 0.05-250 mg, preferably from 0.1-125 mg, of a cyanoguanidine IKK inhibitor compound of formula I and a second anti-neoplastic drug per dosage unit.

The present use also involves non-simultaneous administration of a cyanoguanidine IKK inhibitor compound of formula I and of a second anti-neoplastic drug to a patient. The interval between dosing of the individual compounds as well as the dosing frequency of the individual compounds constituting a combination treatment is determined by many factors including, but not limited to the severity of the disease to be treated, the general condition of the patient, the desired effect and the progress of the treatment. It is within the capability of a skilled physician or veterinary to determine the interval between the individual dosages as well as the dosage frequency, in order to utilise the present invention to its fullest extent.

The neoplastic disease to be treated with a combination of a cyanoguanidine IKK inhibitor compound of formula I and a second anti-neoplastic drug is preferably a malignant disease, i.e. cancer. The cancer disease may e.g. be hematological cancer, such as leukaemia, acute myeloideleukaemia, chronic myeloide leukaemia, chronic lymphatic leukaemia, myelodysplasia, multiple myeloma, Hodgkin's disease or non-Hodgkin's lymphoma or solid tumour cancer, such as fibrosarcom, small or non-small cell lung carcinoma, gastric, intestinal or colorectal cancer, prostate, ovarian or breast cancer, brain, head or neck cancer, cancer in the urinary tract, such as kidney or bladder cancer, malignant melanoma, liver cancer, uterine or pancreatic cancer, etc.

In a preferred embodiment, these cancers are resistant to conventional treatment of neoplastic diseases such as chemotherapeutic agents and hormonal agents, as indicated above.

The invention is further illustrated by the following, non-limiting example.

### EXAMPLES

Experiments were conducted in nude mice inoculated with cancer cells to assess the efficacy of combination therapy using compounds of formula I, *in casu* N-cyano-N'-(6-(4-chlorophenoxy)hexyl)-N"(4-pyridyl)guanidine (hereafter Compound A) and various anti-neoplastic drugs.

Compound A was prepared as disclosed in e.g. EP660 823, WO 94/06770, WO 98/54141 and WO 98/54143.

The following anti-neoplastic drugs were employed in the experiments
- Etoposide is a well-known generic anti-neoplastic drug belonging to the group of antimitotic drugs. Etoposide has significant antitumour activity against *inter alia* germ-cell malignancies, lung cancer, non-Hodgkin's lymphomas, leukemias, Kaposi's sarcoma, neuroblastoma and soft-tissue sarcomas. Vepesid®, available from Bristol-Meyers Squibb, was used as an example of etoposide.
- TNP-470 is an experimental drug from Takeda. It is a fumagilin analogue, and it is believed to exert its effect by inhibiting angiogenesis.
- Cyclophosphamide is a well-known generic anti-neoplastic drug belonging to the group of alkylating agents. It is a broad spectered chemotherapeutic agent administered e.g. to patient with lung cancer. Sendoxan® available from Asta Medica was used as an example of cyclophosphamide
- Cisplatin is a well-known generic anti-neoplastic drug belonging to the group of alkylating agents. It is a broad spectered chemotherapeutic agent administered e.g. to patients with lung cancer, ovarian cancer and testicle cancer. Platinol® available form Bristol Meyers-Squibbs was used as an example of cisplatin.
- Paclitaxel is a well-known generic anti-neoplastic drug belonging to the group of antimitotic agents. It is a broad spectered chemotherapeutic agent administered e.g. to patient with ovarian cancer and non-small cell lung carcinoma cancer. Taxol available form Bristol Meyers-Squibbs was used as an example of paclitaxel.
- Seocalcitol is an experimental drug from LEO Pharma. It is a vitamin D analogue, and it is currently in development as a drug to treat liver cancer.

The following conditions were common to all experiments

The experiments were conducted with NMRI nu/nu female mice (M&B, Ry, Denmark) kept under semi-sterile conditions. The mice were 6 weeks old on arrival, and they were allowed to acclimatise for approximately 3 weeks before the experiments were initiated. The bedding was autoclaved at 121°C for 20 minutes, the food was sterilised by irradiation, and the drinking water was filtered through a 0.2 µm filter. All handling of the mice was done in laminar flow benches.

Before use, all cell cultures were tested negative for mycoplasma infections.

Each experiment employed 40-60 mice which were inoculated in both flanks and left for the tumours to develop. Following that, the mice were divided into groups, each containing 10 mice and thus 18-20 tumors, according to the size of the tumour, *i.e.* large, medium or small. Subsequently, the mice were randomised into the groups shown in Table 1. Unless stated otherwise all doses have been given daily.

**Tabel 1 Dosing regimes**

| Group | Dosing regime |
|---|---|
| 1 | Control |
| 2 | Anti-neoplastic drug |
| 3 | Compound A |
| 4 | Anti-neoplastic drug + after 1 hour compound A |

The tumours were measured using digital callipers before treatment start (=baseline), twice weekly for the duration of the study, and at the end of the study. Tumour area (A) was calculated as the product of the two measured perpendicular diameters; A=d₁xd₂. For mice with only one tumour, the area of this tumour was calculated and used in the statistical analysis. For mice with two tumours, the average of the two tumour areas was calculated and used in the statistical analysis.

For each animal, an individual curve of the tumour areas observed during the study was plotted against time. The trend was an increasing growth rate of the tumours. In order to ensure an approximate linear correspondence between tumour data and time, the observations were transformed by taking the square root of the tumour areas, corresponding to tumour size in one dimension. As a summary measure of the repeated measurements of tumour areas during the study (tumour growth rate), the estimated slope of the individual curves of the square root transformed curves was used [Matthews, Br. Med. J., 300, 230-235, 1990]. The slope should be interpreted as a measure of the tumour growth rate and was estimated using linear regression analysis by time on the observations during the study. Within each group, the mean of the tumour growth rate was calculated.

### Example 1

### Tumour type: NYH

The NYH cell line originates from a patient with small cell lung cancer. The cell line was obtained from Rigshospitalet, Copenhagen, Denmark.
The mice were inoculated with 1x10⁷ NYH cells in 0.2 ml in each flank, and the tumours were left to grow for approximately 4 days. The duration of the dosing was 14 days. The results from the different dosing regimes are tabulated in Table 2.

**Tabel 2**

| Dosing regime | Mean increment/day (mm/day) |
|---|---|
| Etoposide 10 mg/kg p.o. | 0.41 |
| Compound A 15 mg/kg p.o. | 0.34 |
| Combination etoposide + compound A | -0.34 |
| Control | 0.67 |
| | |
| Cyclophosphamide 7 mg/kg p.o. | 0.40 |
| Compound A 15 mg/kg p.o. | 0.26 |
| Combination cyclophosphamide + compound A | -0.04 |
| Control | 0.61 |
| | |
| Cisplatin 5 mg/kg i.p., once weekly | 0.32 |
| Compound A 15 mg/kg p.o. | 0.02 |
| Combination cisplatin + compound A | -0.16 |
| Control | 0.90 |

### Example 2

### Tumour type: HT 1080.

The HT 1080 cell line originates from a patient with human fibrosarcoma, and it was obtained from ATCC.

The mice were inoculated with 2x10⁶ HT 1080 cells in 0.2 ml in each flank, and the tumours were left to grow for approximately 4 days. The duration of the dosing was 14 days. The results from the different dosing regimes are tabulated in Table 3.

**Tabel 3**

| Dosing regime | Mean increment/day (mm/day) |
|---|---|
| Paclitaxel 1 mg/kg s.c. | 0.39 |
| Paclitaxel 2 mg/kg s.c. | 0.15 |
| Compound A 50 mg/kg p.o. | 0.30 |
| Combination paclitaxel 1 mg/kg + compound A | 0.14 |
| Combination paclitaxel 2 mg/kg + compound A | 0.04 |
| Control | 0.67 |
| | |
| Etoposide 10 mg/kg p.o. | -0.01 |
| Compound A 50 mg/kg p.o. | 0.21 |
| Combination etoposide + compound A | -0.12 |
| Control | 0.45 |
| | |
| TNP-470 9 mg/kg s.c. | 0.51 |
| Compound A 50 mg/kg p.o. | 0.14 |
| Combination TNP-470 + compound A | -0.10 |
| Control | 0.73 |
| | |
| Seocalcitol 2 µg/kg p.o. | 0.95 |
| Compound A 50 mg/kg p.o. | 0.24 |
| Combination seocalcitol + compound A | 0.10 |
| Control | 0.98 |

### Example 3

### Tumour type: Colo 320 DM

The Colo 320 DM cell line originates from a patient with human colon cancer, and it was obtained from ATCC

The mice were inoculated with 5x10⁶ Colo 320 DM cells in 0.2 ml in each flank, and the tumours were left to grow for approximately 7 days. The duration of the dosing was 21 days. The results from the different dosing regimes are tabulated in Table 4.

**Tabel 4**

| Dosing regime | Mean increment/day (mm/day) |
|---|---|
| TNP-470 9 mg/kg s.c. | 0.29 |
| Compound A 50 mg/kg p.o. | 0.22 |
| Combination TNP-470 + compound A | -0.07 |
| Control | 0.55 |

As evidenced by the results reported in Tables 2-4, there is a beneficial effect obtained by combining known anti-neoplastic drugs with compounds of formula I, *in casu* N-cyano-N'-(6-(4-chlorophenoxy)hexyl)-N"(4-pyridyl)guanidine. Individual dosing of the two medicaments results in a slower development of the tumour compared to the control. The combination of the two medicaments, however, results in a much slower and in most cases, in fact, an actual decrease in the tumour size, as demonstrated by the negative value of the growth rate. The effect of the combination of anti-neoplastic drugs and compounds of formula I is thus greater than expected from the additive effect of the individual medicaments, i.e. synergistic. The synergistic effect has been demonstrated for a variety of anti-neoplastic drugs and tumour types.

## Claims

1. The use of a cyanoguanidine IKK inhibitor compound of the general formula I 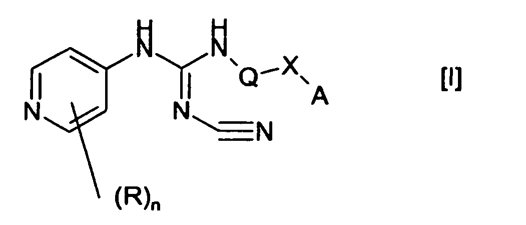 wherein
n is 0, 1 or 2;
each R independently represents halogen, trifluoromethyl, hydroxy, C₁₋₄ alkyl, C₁₋₄ alkoxy, C₁₋₄ alkoxycarbonyl, nitro, sulfo, cyano, amino or carboxy groups;
Q is a straight or branched, saturated or unsaturated C₄₋₂₀ divalent hydrocarbon radical;
X is a bond, amino, O, S, carbonyl, carbonylamino, aminocarbonyl, oxycarbonyloxy, oxycarbonyl, carbonyloxy, aminocarbonyloxy, aminothiocarbonyloxy, oxycarbonylamino or oxythiocarbonylamino;
A is di-(C₁₋₄ alkoxy)phospinoyloxy, C₁₋₄ alkoxycarbonyl, C₁₋₄ alkoxycarbonylamino or C₃₋₁₂ carbocyclic ring optionally substituted with one or more R₁; R₁ being independently selected from the group consisting of halogen, trifluoromethyl, hydroxy, C₁₋₄ alkyl, C₁₋₄ alkoxy, C₁₋₄ alkoxycarbonyl, nitro, cyano, amino, sulfo, carboxy, carboxamido, sulfamoyl or C₁₋₄ hydroxyalkyl;
or a pharmaceutical acceptable salt or N-oxide thereof as a first anti-neoplastic drug for the preparation of a medicament for treatment of neoplastic diseases which, when administered simultaneously or sequentially with a second anti-neoplastic drug, is for prevention of the development of resistance of cancer cells to apoptosis that results from the activation of NFκB by said second anti-neoplastic drug.

2. The use according to claim 1 wherein R is C₁₋₄ alkyl, n being 1.

3. The use according to claim 1 wherein n = 0.

4. The use according to claim 1 wherein A is a phenyl, optionally substituted with a substituent selected from the group consisting of halogen, trifluoromethyl, hydroxy, C₁₋₄ alkyl, C₁₋₄ alkoxy, C₁₋₄ alkoxycarbonyl, nitro, cyano, amino, carboxy, carboxamido, sulfamoyl or C₁₋₄ hydroxyalkyl.

5. The use according to claim 1 wherein A in an optionally substituted pyranyl.

6. The use according to claim 1 wherein X is O.

7. The use according to claim 1 wherein X is amino.

8. The use according to claim 1 wherein Q is a C₄₋₁₂ divalent hydrocarbon radical.

9. The use according to claim 1 wherein the compound of formula I is selected from the group consisting of
N-cyano-N'-(6-(4-chlorophenoxy)hexyl)-N"-(4-pyridyl)guanidine
N-cyano-N'-(7-phenoxyheptyl)-N"-(4-pyridyl) guanidine
N-(12-(*tert*-butoxycarbonylamino)dodecanyl)-N'-cyano-N"-(4-pyridyl) guanidine
N-cyano-N'-(11-(tetrahydropyran-2-yloxy)-undecanyl-N"-(4-pyridyl) guanidine
N-cyano-N'-(6-(2-methoxyphenoxy)hexyl)-N"-(4-pyridyl) guanidine
N-(6-(2,4,5-trichlorophenoxy)hexyl)-N'-cyano-N"-(4-pyridyl) guanidine
N-(6-(1-chlorophenoxy)hexyl))-N'-cyano-N"-(4-pyridyl) guanidine

10. The use according to claim 1, wherein the medicament comprises the first anti-neoplastic drug and the second anti-neoplastic drug in separate containers intended for simultaneous or sequential administration of said anti-neoplastic drugs.

11. The use according to any of claims 1-10, wherein the second anti-neoplastic drug is selected from the group consisting of alkylating agents, antimetabolites, antimitotic agents, antibiotic agents, hormonal agents and antiangiogenic agents.

12. The use according to any of claims 1-11 wherein the first anti-neoplastic drug is N-cyano-N'-(6-(4-chlorophenoxy)hexyl)-N"-(4-pyridyl)guanidine and the anti-neoplastic drug is selected from the group consisting of alkylating agents, antimitotic agents and antiangiogenic agents.

13. The use according to any of claims 1-12 wherein the neoplastic disease is selected from the group consisting of hematological cancer or solid tumour cancer.

14. The use according to claim 13 wherein the neoplastic disease is selected from the group consisting of leukaemia, acute myeloid leukaemia, chronic myeloid leukaemia, chronic lymphatic leukaemia, myelodysplasia, multiple myeloma, Hodgkin's disease or non-Hodgkin's lymphoma, fibrosarcoma, small or non-small cell lung carcinoma, gastric, intestinal or colorectal cancer, prostate, ovarian or breast cancer, head, brain or neck cancer, cancer in the urinary tract, such as kidney or bladder cancer, malignant melanoma, liver cancer, uterine or pancreatic cancer.

15. The use according to any claims 1 to 14, wherein the neoplastic disease or condition exhibits resistance to treatment with anti-neoplastic drugs.

16. A cyanoguandine IKK inhibitor compound of the general formula I 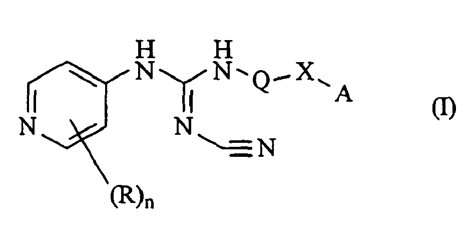 wherein
n is 0, 1 or 2;
each R independently represents halogen, trifluoromethyl, hydroxy, C₁₋₄ alkyl, C₁₋₄ alkoxy, C₁₋₄ alkoxycarbonyl, nitro, sulfo, cyano, amino or carboxy groups;
Q is a straight or branched, saturated or unsaturated C₄₋₂₀ divalent hydrocarbon radical;
X is a bond, amino, O, S, carbonyl, carbonylamino, aminocarbonyl, oxycarbonyloxy, oxycarbonyl, carbonyloxy, aminocarbonyloxy, aminothiocarbonyloxy, oxycarbonylamino or oxythiocarbonylamino;
A is di-(C₁₋₄ alkoxy)phospinoyloxy, C₁₋₄ alkoxycarbonyl, C₁₋₄ alkoxycarbonylamino or C₃₋₁₂ carbocyclic ring optionally substituted with one or more R,; R₁ being independently selected from the group consisting of halogen, trifluoromethyl, hydroxy, C₁₋₄ alkyl, C₁₋₄ alkoxy, C₁₋₄ alkoxycarbonyl, nitro, cyano, amino, sulfo, carboxy, carboxamido, sulfamoyl or C₁₋₄ hydroxyalkyl;
or a pharmaceutically acceptable salt or N-oxide thereof for use as a first anti-neoplastic drug in the treatment of neoplastic diseases, which treatment comprises administering simultaneously or sequentially said compound and a second anti-neoplastic drug, and is for prevention of the development or resistance of cancer cells to apoptosis that results from the activation of NFkB by said second anti-neoplastic drug.

17. The compound according to claim 16, wherein R is C₁₋₄ alkyl, n being 1.

18. The compound according to claim 16, wherein n = 0.

19. The compound according to claim 16, wherein A is a phenyl, optionally substituted with a substituent selected from the group consisting of halogen, trifluoromethyl, hydroxy, C₁₋₄ alkyl, C₁₋₄ alkoxy, C₁₋₄ alkoxycarbonyl, nitro, cyano, amino, carboxy, carboxamido, sulfamoyl or C₁₋₄ hydroxyalkyl.

20. The compound according to claim 16, wherein A is an optionally substituted pyranyl.

21. The compound according to claim 16, wherein X is O.

22. The compound to claim 16, wherein X is amino.

23. The compound according to claim 16, wherein Q is a C₄₋₁₂ divalent hydrocarbon radical.

24. The compound according to claim 16, wherein the compound of formula I is selected from the group consisting of
N-cyano-N'-(6-(4-chlorophenoxy)hexyl)-N"-(4-pyridyl) guanidine
N-cyano-N'-(7-phenoxyheptyl)-N"-(4-pyridyl) guanidine
N-(12-(*tert*-butoxycarbonylamino)dodecanyl)-N'-cyano-N"-(4-pyridyl) guanidine
N-cyano-N'-(11-(tetrahydropyran-2-yloxy)-undecanyl-N"-(4-pyridyl) guanidine
N-cyano-N'-(6-(2-methoxyphenoxy)hexyl)-N"-(4-pyridyl) guanidine
N-(6-(2,4,5-trichlorophenoxy)hexyl)-N'-cyano-N"-(4-pyridyl) guanidine
N-(6-(1-chlorophenoxy)hexyl))-N'-cyano-N"-(4-pyridyl) guanidine.

25. The compound according to claim 16, wherein said compound and the second anti-neoplastic drug are in separate containers intended for simultaneous or sequential administration of said anti-neoplastic drugs.

26. The compound according to any of claims 16 to 25, wherein the second anti-neoplastic drug is selected from the group consisting of alkylating agents, antimetabolites, antimitotic agents, antibiotic agents, hormonal agents and antiangiogenic agents.

27. The compound according to any of claims 16 to 26, wherein said compound is N-cyano-N'-(6-(4-chlorophenoxy)hexyl)-N"-(4-pyridyl) guanidine and the anti-neoplastic drug is selected from the group consisting of alkylating agents, antimitotic agents and antiangiogenic agents.

28. The compound according to any of claims 16 to 27, wherein the neoplastic disease is selected from the group consisting of haematological cancer or solid tumour cancer.

29. The compound according to claim 28, wherein the neoplastic disease is selected from the group consisting of leukaemia, acute myeloid leukaemia, chronic myeloid leukaemia, chronic lymphatic leukaemia, myelodysplasia, multiple myeloma, Hodgkin's disease or non-Hodgkin's lymphoma, fibrosarcoma, small or non-small cell lung carcinoma, gastric intestinal or colorectal cancer, prostate, ovarian or breast cancer, head, brain or neck cancer, cancer in the urinary tract, such as kidney or bladder cancer, malignant melanoma, liver cancer, uterine or pancreatic cancer.

30. The compound according to any of claims 16 to 29, wherein the neoplastic disease or condition exhibits resistance to treatment with anti-neoplastic drugs.

## Patentansprüche

1. Verwendung einer Cyanoguanidin-IKK-Inhibitorverbindung der allgemeinen Formel I 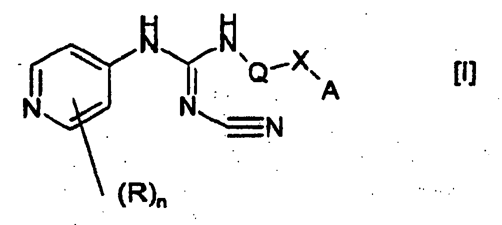 worin
n 0, 1 oder 2 ist,
jedes R unabhängig Halogen-, Trifluormethyl-, Hydroxy-, C₁₋₄-Alkyl-, C₁₋₄-Alkoxy-, C₁₋₄-Alkoxycarbonyl-, Nitro-, Sulfo-, Cyano-, Amino- oder Carboxygruppen darstellt,
Q ein geradkettiger oder verzweigter, gesättigter oder ungesättigter zweiwertiger C₄₋₂₀-Kohlenwasserstoffrest ist,
X eine Bindung, Amino, O, S, Carbonyl, Carbonylamino, Aminocarbonyl, Oxycarbonyloxy, Oxycarbonyl, Carbonyloxy, Aminocarbonyloxy, Aminothiocarbonyloxy, Oxycarbonylamino oder Oxythiocarbonylamino ist,
A Di-(C₁₋₄-alkoxy)phosphinoyloxy, C₁₋₄-Alkoxycarbonyl-, C₁₋₄-Alkoxycarbonylamino oder ein carbocyclischer C₃₋₁₂-Ring ist, der gegebenenfalls mit einem oder mehreren R₁ substituiert ist, wobei R₁ unabhängig aus der Gruppe, bestehend aus Halogen, Trifluormethyl, Hydroxy, C₁₋₄-Alkyl, C₁₋₄-Alkoxy, C₁₋₄-Alkoxycarbonyl, Nitro, Cyano, Amino, Sulfo, Carboxy, Carboxamido, Sulfamoyl oder C₁₋₄-Hydroxyalkyl, ausgewählt ist,
oder eines pharmazeutisch verträglichen Salzes oder N-Oxids davon als ein erster antineoplastischer Arzneistoff für die Herstellung eines Medikaments zur Behandlung neoplastischer Erkrankungen, der, wenn er gleichzeitig oder in einer Abfolge mit einem zweiten antineoplastischen Arzneistoff verabreicht wird, zur Prävention der Entwicklung einer Resistenz von Krebszellen bezüglich einer Apoptose dient, die sich aus der Aktivierung von NFκB durch den zweiten antineoplastischen Arzneistoff ergibt.

2. Verwendung nach Anspruch 1, bei der R C₁₋₄-Alkyl ist und n 1 ist.

3. Verwendung nach Anspruch 1, bei der n = 0.

4. Verwendung nach Anspruch 1, bei der A ein Phenyl ist, das gegebenenfalls mit einem Substituenten substituiert ist, der aus der Gruppe, bestehend aus Halogen, Trifluormethyl, Hydroxy, C₁₋₄-Alkyl, C₁₋₄-Alkoxy, C₁₋₄-Alkoxycarbonyl, Nitro, Cyano, Amino, Carboxy, Carboxamido, Sulfamoyl oder C₁₋₄-Hydroxyalkyl, ausgewählt ist.

5. Verwendung nach Anspruch 1, bei der A ein gegebenenfalls substituiertes Pyranyl ist.

6. Verwendung nach Anspruch 1, bei der X O ist.

7. Verwendung nach Anspruch 1, bei der X Amino ist.

8. Verwendung nach Anspruch 1, bei der Q ein zweiwertiger C₄₋₁₂-Kohlenwasserstoffrest ist.

9. Verwendung nach Anspruch 1, bei der die Verbindung der Formel I aus der Gruppe, bestehend aus
N-Cyano-N'-(6-(4-chlorphenoxy)hexyl)-N"-(4-pyridyl)guanidin,
N-Cyano-N'-(7-phenoxyheptyl)-N "-(4-pyridyl)guanidin,
N-(12-(tert-Butoxycarbonylamino)dodecanyl)-N'-cyano-N"-(4-pyridyl)guanidin,
N-Cyano-N'-(11-(tetrahydropyran-2-yloxy)-undecanyl-N"-(4-pyridyl)guanidin,
N-Cyano-N'-(6-(2-methoxyphenoxy)hexyl)-N"-(4-pyridyl)guanidin,
N-(6-(2,4,5-Trichlorphenoxy)hexyl)-N'-cyano-N "-(4-pyridyl)guanidin,
N-(6-(1-Chlorphenoxy)hexyl)-N'-cyano-N''-(4-pyridyl)guanidine,
ausgewählt ist.

10. Verwendung nach Anspruch 1, bei der das Medikament den ersten antineoplastischen Arzneistoff und den zweiten antineoplastischen Arzneistoff in separaten Behältern enthält, die für eine gleichzeitige oder aufeinander folgende Verabreichung der antineoplastischen Arzneistoffe vorgesehen sind.

11. Verwendung nach einem der Ansprüche 1 bis 10, bei welcher der zweite antineoplastische Arzneistoff aus der Gruppe, bestehend aus Alkylierungsmitteln, Antimetaboliten, Antimitotika, antibiotischen Mitteln, hormonellen Mitteln und Antiangiogenesemitteln, ausgewählt ist.

12. Verwendung nach einem der Ansprüche 1 bis 11, bei welcher der erste antineoplastische Arzneistoff N-Cyano-N'-(6-(4-chlorphenoxy)hexyl)-N"-(4-pyridyl)guanidin ist und der antineoplastische Arzneistoff aus der Gruppe, bestehend aus Alkylierungsmitteln, Antimitotika und Antiangiogenesemittein ausgewählt ist.

13. Verwendung nach einem der Ansprüche 1 bis 12, bei der die neoplastische Erkrankung aus der Gruppe, bestehend aus hämatologischem Krebs oder Krebs mit solidem Tumor, ausgewählt ist.

14. Verwendung nach Anspruch 13, bei der die neoplastische Erkrankung aus der Gruppe, bestehend aus Leukämie, akuter myeloider Leukämie, chronischer myeloider Leukämie, chronischer lymphatischer Leukämie, Myelodysplasie, multiplem Myelom, Hodgkin-Krankheit oder nicht-Hodgkin-Lymphom, Fibrosarkom, kleinzelligem oder nicht-kleinzelligem Lungenkarzinom, Magen-, Darm- oder kolorektalem Krebs, Prostata-, Eierstock- oder Brustkrebs, Kopf-, Gehirn- oder Halskrebs, Krebs im Harntrakt, wie z.B. Nieren- oder Blasenkrebs, malignem Melanom, Leberkrebs, Gebärmutter oder Pankreaskrebs, ausgewählt ist.

15. Verwendung nach einem der Ansprüche 1 bis 14, bei der die neoplastische Erkrankung oder der neoplastische Zustand eine Resistenz gegen eine Behandlung mit antineoplastischen Arzneistoffen zeigt.

16. Cyanoguanidin-IKK-Inhibitorverbindung der allgemeinen Formel I 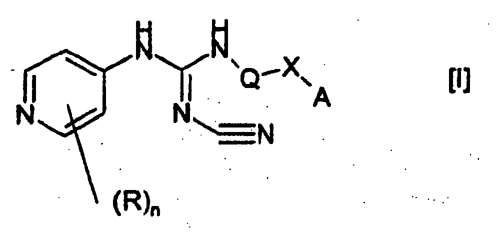 worin
n 0, 1 oder 2 ist,
jedes R unabhängig Halogen-, Trifluormethyl-, Hydroxy-, C₁₋₄-Alkyl-, C₁₋₄-Alkoxy-, C₁₋₄-Alkoxycarbonyl-, Nitro-, Sulfo-, Cyano-, Amino- oder Carboxygruppen darstellt,
Q ein geradkettiger oder verzweigter, gesättigter oder ungesättigter zweiwertiger C₄₋₂₀-Kohlenwasserstoffrest ist,
X eine Bindung, Amino, O, S, Carbonyl, Carbonylamino, Aminocarbonyl, Oxycarbonyloxy, Oxycarbonyl, Carbonyloxy, Aminocarbonyloxy, Aminothiocarbonyloxy, Oxycarbonylamino oder Oxythiocarbonylamino ist,
A Di-(C₁₋₄-alkoxy)phosphinoyloxy, C₁₋₄-Alkoxycarbonyl-, C₁₋₄-Alkoxycarbonylamino oder ein carbocyclischer C₃₋₁₂-Ring ist, der gegebenenfalls mit einem oder mehreren R₁ substituiert ist, wobei R₁ unabhängig aus der Gruppe, bestehend aus Halogen, Trifluormethyl, Hydroxy, C₁₋₄-Alkyl, C₁₋₄-Alkoxy, C₁₋₄-Alkoxycarbonyl, Nitro, Cyano, Amino, Sulfo, Carboxy, Carboxamido, Sulfamoyl oder C₁₋₄-Hydroxyalkyl, ausgewählt ist,
oder ein pharmazeutisch verträgliches Salz oder N-Oxid davon zur Verwendung als ein erster antineoplastischer Arzneistoff zur Behandlung neoplastischer Erkrankungen, wobei die Behandlung das gleichzeitige oder aufeinander folgende Verabreichen der Verbindung und eines zweiten antineoplastischen Arzneistoffs umfasst und zur Prävention der Entwicklung einer Resistenz von Krebszellen bezüglich einer Apoptose dient, die sich aus der Aktivierung von NFκB durch den zweiten antineoplastischen Arzneistoff ergibt.

17. Verbindung nach Anspruch 16, bei der R C₁₋₄-Alkyl ist und n 1 ist.

18. Verbindung nach Anspruch 16, bei der n = 0.

19. Verbindung nach Anspruch 16, bei der A ein Phenyl ist, das gegebenenfalls mit einem Substituenten substituiert ist, der aus der Gruppe, bestehend aus Halogen, Trifluormethyl, Hydroxy, C₁₋₄-Alkyl, C₁₋₄-Alkoxy, C₁₋₄-Alkoxycarbonyl, Nitro, Cyano, Amino, Carboxy, Carboxamido, Sulfamoyl oder C₁₋₄-Hydroxyalkyl, ausgewählt ist.

20. Verbindung nach Anspruch 16, bei der A ein gegebenenfalls substituiertes Pyranyl ist.

21. Verbindung nach Anspruch 16, bei der X O ist.

22. Verbindung nach Anspruch 16, bei der X Amino ist.

23. Verbindung nach Anspruch 16, bei der Q ein zweiwertiger C₄₋₁₂-Kohlenwasserstoffrest ist.

24. Verbindung nach Anspruch 16, wobei die Verbindung der Formel I aus der Gruppe, bestehend aus
N-Cyano-N'-(6-(4-chlorphenoxy)hexyl)-N"-(4-pyridyl)guanidin,
N-Cyano-N'-(7-phenoxyheptyl)-N"-(4-pyridyl)guanidin,
N-(12-(tert-Butoxycarbonylamino)dodecanyl)-N'-cyano-N"-(4-pyridyl)guanidin,
N-Cyano-N'-(11-(tetrahydropyran-2-yloxy)-undecanyl-N"-(4-pyridyl)guanidin,
N-Cyano-N'-(6-(2-methoxyphenoxy)hexyl)-N "-(4-pyridyl)guanidin,
N-(6-(2,4,5-Trichlorphenoxy)hexyl)-N'-cyano-N"-(4-pyridyl)guanidin,
N-(6-(1-Chlorphenoxy)hexyl)-N'-cyano-N"-(4-pyridyl)guanidine,
ausgewählt ist.

25. Verbindung nach Anspruch 16, wobei die Verbindung und der zweite antineoplastische Arzneistoff in separaten Behältern vorliegen, die für eine gleichzeitige oder aufeinander folgende Verabreichung der antineoplastischen Arzneistoffe vorgesehen sind.

26. Verbindung nach einem der Ansprüche 16 bis 25, wobei der zweite antineoplastische Arzneistoff aus der Gruppe, bestehend aus Alkylierungsmitteln, Antimetaboliten, Antimitotika, antibiotische Mittel, hormonelle Mittel und Antiangiogenesemitteln, ausgewählt ist.

27. Verbindung nach einem der Ansprüche 16 bis 26, wobei die Verbindung N-Cyano-N'-(6-(4-chlorphenoxy)hexyl)-N"-(4-pyridyl)guanidin ist und der antineoplastische Arzneistoff aus der Gruppe, bestehend aus Alkylierungsmitteln, Antimitotika und Antiangiogenesemitteln ausgewählt ist.

28. Verbindung nach einem der Ansprüche 16 bis 27, wobei die neoplastische Erkrankung aus der Gruppe, bestehend aus hämatologischem Krebs oder Krebs mit solidem Tumor, ausgewählt ist.

29. Verbindung nach Anspruch 28, wobei die neoplastische Erkrankung aus der Gruppe, bestehend aus Leukämie, akuter myeloider Leukämie, chronischer myeloider Leukämie, chronischer lymphatischer Leukämie, Myelodysplasie, multiplem Myelom, Hodgkin-Krankheit oder nicht-Hodgkin-Lymphom, Fibrosarkom, kleinzelligem oder nicht-kleinzelligem Lungenkarzinom, Magen-, Darm- oder kolorektalem Krebs, Prostata-, Eierstock- oder Brustkrebs, Kopf-, Gehirn- oder Halskrebs, Krebs im Harntrakt, wie z.B. Nieren- oder Blasenkrebs, malignem Melanom, Leberkrebs, Gebärmutter oder Pankreaskrebs, ausgewählt ist.

30. Verbindung nach einem der Ansprüche 16 bis 29, wobei die neoplastische Erkrankung oder der neoplastische Zustand eine Resistenz gegen eine Behandlung mit antineoplastischen Arzneistoffen zeigt.

## Revendications

1. Utilisation d'un composé inhibiteur d'IKK de type cyanoguanidine de formule générale I 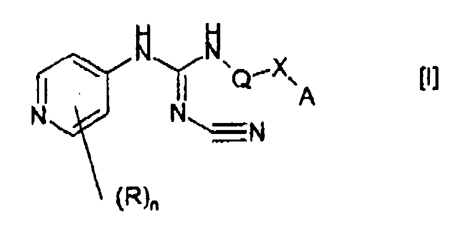 dans laquelle
n est 0, 1 ou 2 ;
chaque R représente indépendamment un halogène, un groupe trifluorométhyle, hydroxy, alkyle en C₁₋₄, alcoxy en C₁₋₄, alcoxycarbonyle en C₁₋₄, nitro, sulfo, cyano, amino ou carboxy ;
Q est un radical hydrocarboné divalent en C₄₋₂₀ linéaire ou ramifié, saturé ou insaturé ;
X est une liaison, un amino, O, S, un carbonyle, un carbonylamino, un aminocarbonyle, un oxycarbonyloxy, un oxycarbonyle, un carbonyloxy, un aminocarbonyloxy, un aminothiocarbonyloxy, un oxycarbonylamino ou un oxythiocarbonylamino ;
A est un di(alcoxy en C₁₋₄)phosphinoyloxy, un alcoxycarbonyle en C₁₋₄, un alcoxycarbonylamino en C₁₋₄ ou un cycle carbocyclique en C₃₋₁₂ facultativement substitué par un ou plusieurs R₁ ; R₁ étant indépendamment choisi parmi un halogène, un trifluorométhyle, un hydroxy, un alkyle en C₁₋₄, un alcoxy en C₁₋₄, un alcoxycarbonyle en C₁₋₄, un nitro, un cyano, un amino, un sulfo, un carboxy, un carboxyamido, un sulfamoyle ou un hydroxyalkyle en C₁₋₄ ;
ou d'un sel ou N-oxyde acceptable du point de vue pharmaceutique de celui-ci en tant que premier médicament antinéoplasique pour la préparation d'un médicament pour le traitement de maladies néoplasiques lequel, lorsqu'il est administré de façon simultanée ou séquentielle avec un second médicament antinéoplasique, sert à la prévention du développement de la résistance de cellules cancéreuses à l'apoptose qui résulte de l'activation du NFκB par ledit second médicament antinéoplasique.

2. Utilisation selon la revendication 1 dans laquelle R est un alkyle en C₁₋₄, n valant 1.

3. Utilisation selon la revendication 1 dans laquelle n = 0.

4. Utilisation selon la revendication 1 dans laquelle A est un phényle, facultativement substitué par un substituant choisi dans le groupe constitué des groupes halogène, trifluorométhyle, hydroxy, alkyle en C₁₋₄, alcoxy en C₁₋₄, alcoxycarbonyle en C₁₋₄, nitro, cyano, amino, carboxy, carboxyamido, sulfamoyle ou hydroxyalkyle en C₁₋₄.

5. Utilisation selon la revendication 1 dans laquelle A est un pyranyle facultativement substitué.

6. Utilisation selon la revendication 1 dans laquelle X est O.

7. Utilisation selon la revendication 1 dans laquelle X est un amino.

8. Utilisation selon la revendication 1 dans laquelle Q est un radical hydrocarboné divalent en C₄₋₁₂.

9. Utilisation selon la revendication 1 dans laquelle le composé de formule I est choisi parmi
la *N*-cyano-*N'*-(6-(4-chlorophénoxy)hexyl)-*N"*-(4-pyridyl)guanidine,
la *N*-cyano-*N*'-(7-phénoxyheptyl)-*N*"-(4-pyridyl)guanidine,
la *N*-(12-(*tert*-butoxycarbonylamino)dodécanyl)-*N'*-cyano-*N"*-(4-pyridyl)guanidine,
la *N*-cyano-*N'*-(11-(tétrahydropyran-2-yloxy)undécanyl-*N"*-(4-pyridyl)guanidine,
la *N*-cyano-*N'*-(6-(2-méthoxyphénoxy)hexyl)-*N"*-(4-pyridyl)guanidine,
la *N*-6-(2,4,5-trichlorophénoxy)hexyl)-*N'*-cyano-*N"*-(4-pyridyl)guanidine,
la *N*-6-(1-chlorophénoxy)hexyl)-*N'*-cyano-*N"*-(4-pyridyl)guanidine.

10. Utilisation selon la revendication 1, dans laquelle le médicament comprend le premier médicament antinéoplasique et le second médicament antinéoplasique dans des récipients séparés destinés à l'administration simultanée ou séquentielle desdits médicaments antinéoplasiques.

11. Utilisation selon l'une quelconque des revendications 1-10, dans laquelle le second médicament antinéoplasique est choisi parmi des agents alkylants, des antimétabolites, des agents antimitotiques, des agents antibiotiques, des agents hormonaux et des agents antiangiogéniques.

12. Utilisation selon l'une quelconque des revendications 1-11 dans laquelle le premier médicament antinéoplasique est la *N*-cyano-*N*'-6-(4-chlorophénoxy)hexyl)-*N"*-(4-pyridyl)guanidine et le médicament antinéoplasique est choisi parmi des agents alkylants, des agents antimitotiques et des agents antiangiogéniques.

13. Utilisation selon l'une quelconque des revendications 1-12 dans laquelle la maladie néoplasique est choisie entre un cancer hématologique ou un cancer à tumeur solide.

14. Utilisation selon la revendication 13 dans laquelle la maladie néoplasique est choisie parmi la leucémie, la leucémie myéloïde aiguë, la leucémie myéloïde chronique, la leucémie lymphoïde chronique, la myélodysplasie, le myélome multiple, la maladie de Hodgkin ou le lymphome non Hodgkinien, le fibrosarcome, le cancer des poumons à petites cellules ou à grandes cellules, le cancer de l'estomac, de l'intestin ou colorectal, le cancer de la prostate, des ovaires ou du sein, le cancer de la tête, du cerveau ou du cou, le cancer dans le tractus urinaire tel que le cancer du rein ou de la vessie, le mélanome malin, le cancer du foie, le cancer de l'utérus ou du pancréas.

15. Utilisation selon l'une quelconque des revendications 1 à 14, dans laquelle la maladie ou affection néoplasique présente une résistance au traitement avec des médicaments antinéoplasiques.

16. Composé inhibiteur d'IKK de type cyanoguanidine de formule générale I 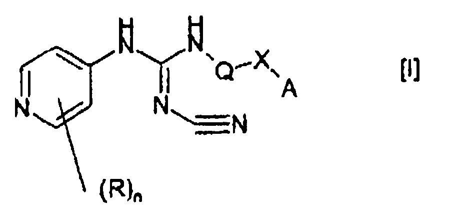 dans laquelle
n est 0, 1 ou 2;
chaque R représente indépendamment un halogène, un groupe trifluorométhyle, hydroxy, alkyle en C₁₋₄, alcoxy en C₁₋₄, alcoxycarbonyle en C₁₋₄, nitro, sulfo, cyano, amino ou carboxy ;
Q est un radical hydrocarboné divalent en C₄₋₂₀ linéaire ou ramifié, saturé ou insaturé ;
X est une liaison, un amino, 0, S, un carbonyle, un carbonylamino, un aminocarbonyle, un oxycarbonyloxy, un oxycarbonyle, un carbonyloxy, un aminocarbonyloxy, un aminothiocarbonyloxy, un oxycarbonylamino ou un oxythiocarbonylamino ;
A est un di(alcoxy en C₁₋₄)phosphinoyloxy, un alcoxycarbonyle en C₁₋₄, un alcoxycarbonylamino en C₁₋₄ ou un cycle carbocyclique en C₃₋₁₂ facultativement substitué par un ou plusieurs R₁ ; R₁ étant indépendamment choisi parmi un halogène, un trifluorométhyle, un hydroxy, un alkyle en C₁₋₄, un alcoxy en C₁₋₄, un alcoxycarbonyle en C₁₋₄, un nitro, un cyano, un amino, un sulfo, un carboxy, un carboxyamido, un sulfamoyle ou un hydroxyalkyle en C₁₋₄ ;
ou sel ou N-oxyde acceptable du point de vue pharmaceutique de celui-ci destiné à être utilisé en tant que premier médicament antinéoplasique dans le traitement de maladies néoplasiques, lequel traitement comprend d'administrer de façon simultanée ou séquentielle ledit composé et un second médicament antinéoplasique et sert à la prévention du développement de la résistance de cellules cancéreuses à l'apoptose qui résulte de l'activation du NFκB par ledit second médicament antinéoplasique.

17. Composé selon la revendication 16, dans lequel R est un alkyle en C₁₋₄, n valant 1.

18. Composé selon la revendication 16, dans lequel n = 0.

19. Composé selon la revendication 16, dans lequel A est un phényle, facultativement substitué par un substituant choisi parmi un halogène, un trifluorométhyle, un hydroxy, un alkyle en C₁₋₄, un alcoxy en C₁₋₄, un alcoxycarbonyle en C₁₋₄, un nitro, un cyano, un amino, un carboxy, un carboxyamido, un sulfamoyle ou un hydroxyalkyle en C₁₋₄.

20. Composé selon la revendication 16, dans lequel A est un pyranyle facultativement substitué.

21. Composé selon la revendication 16, dans lequel X est O.

22. Composé selon la revendication 16, dans lequel X est un amino.

23. Composé selon la revendication 16, dans lequel Q est un radical hydrocarboné divalent en C₄₋₁₂.

24. Composé selon la revendication 16, ledit composé de formule I étant choisi parmi
la *N*-cyano-*N'*-6-(4-chlorophénoxy)hexyl)-*N"*-(4-pyridyl)guanidine,
la *N*-cyano-*N'*-(7-phénoxyheptyl)-*N"*-(4-pyridyl)guanidine,
la *N*-(12-(*tert*-butoxycarbonylamino)dodécanyl)-*N'*-cyano-*N"*-(4-pyridyl)guanidine,
la *N*-cyano-*N'*-(11-(tétrahydropyran-2-yloxy)undécanyl-*N"*-(4-pyridyl)guanidine,
la *N*-cyano-*N'*-(6-(2-méthoxyphénoxy)hexyl)-*N"*-(4-pyridyl)guanidine,
la *N*-(6-(2,4,5-trichlorophénoxy)hexcyl)-*N'*-cyano-*N"*-(4-pyridyl)guanidine,
la *N*-(6-(1-chtorophénoxy)hexyt)-*N'*-cyano-*N"*-(4-pyridyl)guanidine.

25. Composé selon la revendication 16, dans lequel ledit composé et le second médicament antinéoplasique sont dans des récipients séparés destinés à l'administration simultanée ou séquentielle desdits médicaments antinéoplasiques.

26. Composé selon l'une quelconque des revendications 16 à 25, dans lequel le second médicament antinéoplasique est choisi parmi des agents alkylants, des antimétabolites, des agents antimitotiques, des agents antibiotiques, des agents hormonaux et des agents antiangiogéniques.

27. Composé selon l'une quelconque des revendications 16 à 26, dans lequel ledit composé est la *N*-cyano-*N'*-6-(4-chlorophénoxy)hexyl)-*N"*-{4-pyridyl)guanidine et le médicament antinéoplàsique est choisi parmi des agents alkylants, des agents antimitotiques et des agents antiangiogéniques.

28. Composé selon l'une quelconque des revendications 16 à 27, dans lequel la maladie néoplasique est choisie entre un cancer hématologique ou un cancer à tumeur solide.

29. Composé selon la revendication 28, dans lequel la maladie néoplasique est choisie parmi la leucémie, la leucémie myéloïde aiguë, la leucémie myéloïde chronique, la leucémie lymphoïde chronique, la myélodysplasie, le myélome multiple, la maladie de Hodgkin ou le lymphome non Hodgkinien, le fibrosarcome, le cancer des poumons à petites cellules ou à grandes cellules, le cancer de l'estomac, de l'intestin ou colorectal, le cancer de la prostate, des ovaires ou du sein, le cancer de la tête, du cerveau ou du cou, le cancer dans le tractus urinaire tel que le cancer du rein ou de la vessie, le mélanome malin, le cancer du foie, le cancer de l'utérus ou du pancréas.

30. Composé selon l'une quelconque des revendications 16 à 29, dans lequel la maladie ou affection néoplasique présente une résistance au traitement avec des médicaments antinéoplasiques.
